# EUROPÄISCHE PATENTSCHRIFT

(11) **EP 0 898 980 B1**
(45) Veröffentlichungstag und Bekanntmachung des Hinweises auf die Patenterteilung: **15.10.2003**
(21) Anmeldenummer: 97202661.1
(22) Anmeldetag: 29.08.1997
(51) Int. Cl.: A61M 25/00

(54) **Medizinischer Katheter**
Medical catheter
Cathéter medical

(43) Veröffentlichungstag der Anmeldung: 03.03.1999
(73) Patentinhaber: Schneider (Europe) GmbH, 8180 Bülach (CH)
(72) Erfinder: Wiesendanger, Hans, 8264 Eschenz (CH)
(74) Vertreter: Kiliaridis, Constantin

(56) Entgegenhaltungen:
- EP-A- 0 298 634
- EP-A- 0 456 342
- EP-A- 0 803 264
- US-A- 4 940 179
- US-A- 5 527 281
- US-A- 5 653 691

## Beschreibung

Die Erfindung bezieht sich auf einen Katheter mit einer ersten Komponente, einer zweiten Komponente und einer zwischen den Komponenten angeordneten Klebfuge, die durch eine die Komponenten verbindende Klebstoffschicht ausgefüllt ist.

Bei der Herstellung von Kathetern für medizinische Interventionen, wie beispielsweise von Ballon- oder Führungskathetern für eine perkutane transluminale Angioplastie oder von Platzierinstrumenten für die Implantation einer Endoprothese, findet eine Vielzahl verschiedener Werkstoffe Anwendung. Dabei werden hauptsächlich Kunststoffe verwendet, wie zum Beispiel Polyamid, Polyimid, Polyetheretherketon für Katheterschäfte, Polycarbonat für Anschlussteile, Nylon oder Polyethylenterephthalat für Dilatationsballons und Polyurethan für Katheterspitzen, aber auch metallische Werkstoffe, wie etwa Nickel-Titan-Legierungen für steifere Schaftabschnitte, Gold für röntgendichte Markierungsringe oder rostfreier Stahl für Versteifungsdrähte. Je nach den für die Funktion erforderlichen physikalischen Eigenschaften ist für jede Katheterkomponente ein geeignetes Material auszuwählen, so dass ein fertig montierter Katheter Verbindungsstellen mit unterschiedlichsten zu fügenden Materialpaarungen aufweist. Die Verbindungen sind im praktischen Einsatz zum Teil starken mechanischen Belastungen ausgesetzt und müssen dabei dem hohen Sicherheitsstandard in der medizinischen Kathetertechnik genügen. Neben dem Aufziehen von Schrumpfschläuchen und dem Verschweissen ist das Kleben von Katheterkomponenten eine relativ einfache und daher gängige Fügetechnik.

Ein derartiger Katheter ist z.B. aus dem Dokument US-A-5 527 281 bekannt.

Mit der grossen Auswahl an vorhandenen Klebstoffen können die meisten Verbindungen zwischen den Komponenten eines Katheters schnell und mit ausreichender Festigkeit hergestellt werden. Die Klebbarkeit von zwei Katheterkomponenten hängt nun davon ab, ob ein Klebstoff zur Verfügung steht, der beim Verfestigen neben der inneren Kohäsionskraft auch Adhäsionskräfte zu den Klebflächen der beiden zu verbindenden Komponenten ausbildet. Wegen der Unterschiedlichkeit der Komponentenmaterialien hinsichtlich ihres chemischen Aufbaus, der die Ausbildung der Haftungskräfte ermöglicht, oder hinsichtlich ihrer Benetzbarkeit kommt es vor, dass sich kein Klebstoff mit beiden Katheterkomponenten gleichzeitig ausreichend fest verbindet. Andererseits ist es möglich, dass bei einem Klebstoff, welcher sich mit beiden Komponenten verbinden würde, der Aushärtungsprozess beispielsweise durch konstruktive Vorgaben nicht durchgeführt werden kann oder zu lange dauern würde. Für die Kathetertechnik bedeutet dies eine Einschränkung in der Materialauswahl beziehungsweise die Anwendung aufwendigerer Verbindungstechniken als Kleben.

Der Erfindung liegt daher die Aufgabe zugrunde, einen Katheter der eingangs genannten Art anzugeben, bei dem die vorgenannten Einschränkungen und Nachteile überwunden sind. Insbesondere sollen Komponenten eines Katheters auch dann fest miteinander verbunden sein, wenn deren Materialien in dem oben beschriebenen Sinne schlecht klebbar sind, aber dennoch eine Klebetechnik angewendet werden soll.

Die Aufgabe wird gemäss der Erfindung gelöst durch einen Katheter der eingangs genannten Art mit den Merkmalen des kennzeichnenden Teils des Patentanspruchs 1. Wenn die Klebstoffschicht zweilagig ausgebildet ist, wobei eine erste Klebstofflage mit der ersten Komponente und eine zweite Klebstofflage mit der ersten Klebstofflage und der zweiten Komponente verbunden ist, sind auch Katheterkomponenten fest miteinander verklebbar, die bei Anwendung einer einlagigen Klebstoffschicht entweder überhaupt nicht oder nur mit unzureichender Festigkeit fügbar wären. Ausserdem kann es vorteilhaft sein, eine erste Klebstofflage beispielsweise im unmontierten Zustand der ersten Komponente zu applizieren und die Verbindung mit einer zweiten Komponente durch die zweite Klebstofflage im zusammengebauten Endzustand herzustellen.

In einer bevorzugten Ausführungsform der Erfindung sind die Klebstofflagen aus chemisch reagierenden Klebstoffen gebildet, da zum Herstellen der Verbindung kein hoher Druck erforderlich ist, sondern im Regelfall das Fixieren der Klebflächen der zu verbindenden Komponenten genügt. Ausserdem gibt es eine grosse Auswahl an chemisch reaktiven Klebstoffen auch mit unterschiedlichen Reaktionsmechanismen.

In vorteilhaften Ausgestaltungen der Erfindung weisen die Klebstoffe unterschiedliche Reaktionsmechanismen auf, da dann auch feste Verbindungen zwischen Katheterkomponenten mit stark unterschiedlichen Klebbarkeitseigenschaften bewerkstelligt werden können. Vorzugsweise reagiert einer der Klebstoffe durch Zugabe von Feuchtigkeit während der andere Klebstoff durch Energiezufuhr reagiert. So kann beispielsweise eine erste, durch Zufuhr von Wärme- oder Lichtstrahlung aushärtende Klebstofflage auf eine erste, strahlungsresistente Komponente im unmontierten Zustand aufgetragen werden, ohne die andere Komponente dabei zu beschädigen, und die Verbindung mit der zweiten Komponente des Katheters durch eine zweite, durch die Feuchte auf der Oberfläche der zu fügenden Komponenten härtende Klebstoffschicht mit hoher Festigkeit im endmontierten Zustand hergestellt werden.

In einer vorteilhaften Ausführungsform der Erfindung ist der durch Zugabe von Feuchtigkeit reagierende Klebstoff ein Cyanacrylat und der durch Zufuhr von Energie reagierende Klebstoff durch Bestrahlung mit ultraviolettem Licht aushärtbar. Beide Klebstoffe sind schnellhärtend und erlauben entsprechend kurze Produktionszeiten für einen erfindungsgemässen Katheter.

Weitere Vorteile eines erfindungsgemässen Katheters ergeben sich aus einem bevorzugten Ausführungsbeispiel, das im folgenden anhand der Zeichnung näher beschrieben wird, in deren
- FIG. 1: der distale Abschnitt einer Stentplatziervorrichtung gemäss der Erfindung im Teilschnitt und in
- FIG. 2: eine Verbindungsstelle zwischen Komponenten der Vorrichtung aus FIG. 1 im Längsschnitt
veranschaulicht sind.

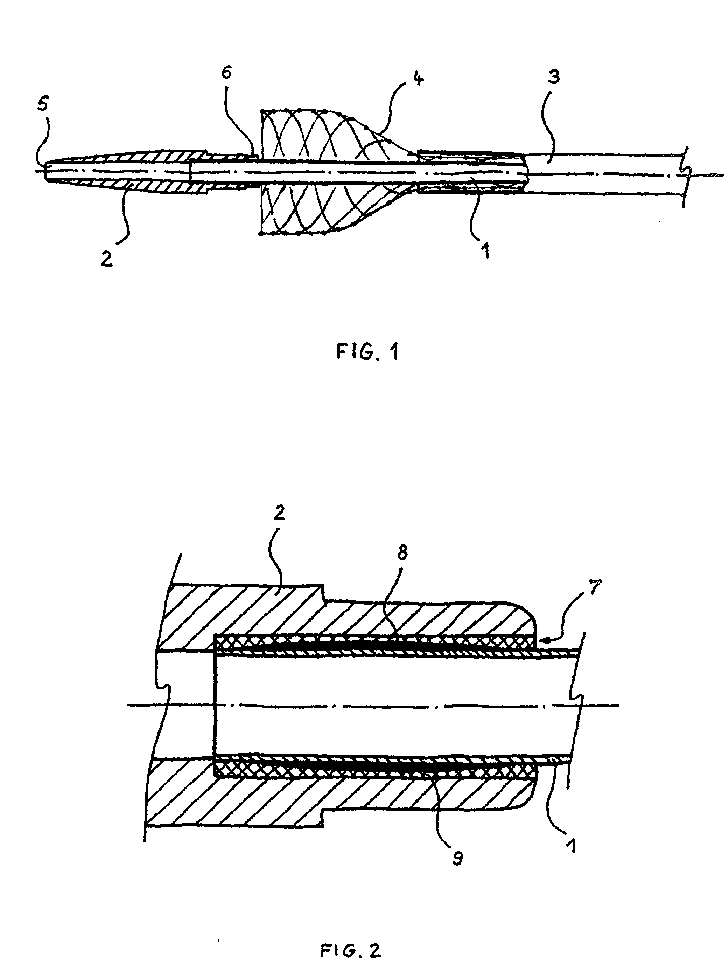

In FIG. 1 ist als Beispiel für einen medizinischen Katheter eine bekannte Stentplatziervorrichtung dargestellt, mit deren Hilfe eine selbstexpandierende Gefässstütze, auch Stent genannt, durch eine Punktionsöffnung in das Blutgefässsystem eingeführt, bis zu ihrer Implantationsstelle vorgeschoben und dort schliesslich freigesetzt wird. Dabei expandiert der Stent durch seine Eigenelastizität und legt sich von innen an die zu stützende Gefässwand an.

Die Stentplatziervorrichtung weist gemäss FIG. 1 einen Innenschaft 1 mit Spitze 2 und einen Aussenschaft 3 auf, welche ineinander angeordnet und in axialer Richtung gegeneinander verschiebbar sind. Der Stent 4 wird zum Einführen radial komprimiert und zwischen Innenschaft 1 und Aussenschaft 2 geladen, wobei der Aussenschaft 3 von proximal auf die Spitze 2 geschoben wird und zu dieser einen glatten, atraumatische Übergang bildet. Die hohl ausgestaltete Spitze 2 bildet mit dem ebenfalls hohlen Innenschaft 1 ein sich über die gesamte Länge des Katheters erstreckendes Führungsdrahtlumen 5 zur Aufnahme eines Führungsdrahtes (nicht dargestellt). Mit der sich verjüngenden Spitze 2 voran wird die Vorrichtung auf dem vorplatzierten Führungsdraht bis zur Implantationsstelle vorgeschoben, wobei die Position der Vorrichtung im Gefässsystem mit Hilfe eines röntgendichten Markierungsringes 6 fluoroskopisch sichtbar gemacht werden kann. Durch Zurückziehen des Aussenschaftes 3 relativ zum Innenschaft 1 wird der selbstexpandierende Stent 4, wie dargestellt, freigesetzt.

Bei all diesen Manipulationen und insbesondere auch beim Zurückziehen des Katheters durch enge und verwundene Gefässe müssen alle Komponentenverbindungen, insbesondere auch die zwischen Innenschaft 1 und Spitze 2, eine ausreichende Festigkeit besitzen, um beispielsweise der Gefahr ihres Verlusts im menschlichen Körper vorzubeugen. Während die Spitze 2 vorzugsweise aus verhältnismässig weichem Polyurethan hergestellt wird und im allgemeinen gute Klebbarkeitseigenschaften aufweist, besteht der Innenschaft 1 aus einem steiferen Werkstoff, wie Polyetheretherketon, welcher aufgrund seiner schlechten Benetzbarkeit nur bedingt klebbar ist. Mit einem UV-härtenden Klebstoff könnte man zwar eine ausreichende Festigkeit der Verbindung erzielen, jedoch kann ein solcher Klebstoff hier nicht eingesetzt werden, da die Spitze 2 aufgrund eines röntgendichten Zusatzstoffes für ultraviolettes Licht nicht durchlässig ist.

Gemäss FIG. 2 ist die Klebfuge zwischen dem Innenschaft 1 und der Spitze 2 von einer zweilagigen Klebstoffschicht 7 ausgefüllt, wobei die erste Klebstofflage 8 mit dem Innenschaft 1 und die zweite Klebstofflage 9 mit der ersten Klebstofflage 8 und der Spitze 2 verbunden ist. Die erste Klebstofflage 8 besteht aus einem UV-härtenden Klebstoff, der auf den Innenschaft 1 aufgetragen und im unmontierten Zustand ausgehärtet wird, da die Klebstelle dann noch zugänglich ist für die Bestrahlung mit ultraviolettem Licht. Anschliessend wird als zweite Klebstofflage 9 ein Cyanacrylat auf die erste Klebstofflage 8 und/oder auf die Klebfläche der Spitze 2 aufgetragen, welche im zusammengesteckten Zustand durch die Oberflächenfeuchte an der gesamten Klebfläche aushärtet. Durch die zweilagige Klebstoffschicht 7 kann die Festigkeit der Verbindung gegenüber einen einlagigen Cyanacrylat-Schicht um ein Mehrfaches erhöht werden.

### Bezugszeichenliste

- 1: erste Komponente, Innenschaft
- 2: zweite Komponente, Spitze
- 3: Aussenschaft
- 4: Stent
- 5: Führungsdrahtlumen
- 6: Markierungsring
- 7: Klebstoffschicht
- 8: erste Klebstofflage
- 9: zweite Klebstofflage

## Patentansprüche

1. Katheter mit einer ersten Komponente (1), einer zweiten Komponente (2) und einer zwischen den Komponenten (1; 2) angeordneten Klebfuge, die durch eine die Komponenten (1; 2) verbindende Klebstoffschicht (7) ausgefüllt ist, **dadurch gekennzeichnet, dass** die Klebstoffschicht (7) zweilagig ausgebildet ist, wobei eine erste Klebstofflage (8) mit der ersten Komponente (1) und eine zweite Klebstofflage (9) mit der ersten Klebstofflage (8) und der zweiten Komponente (2) verbunden ist.

2. Katheter nach Anspruch 1,
**dadurch gekennzeichnet, dass** die Klebstofflagen (8; 9) durch chemisch reagierende Klebstoffe gebildet werden.

3. Katheter nach Anspruch 2,
**dadurch gekennzeichnet, dass** die Klebstofflagen (8; 9) durch chemisch reagierende Klebstoffe mit unterschiedlichen Reaktionsmechanismen gebildet werden.

4. Katheter nach Anspruch 3,
**dadurch gekennzeichnet, dass** ein Klebstoff durch Zugabe von Feuchtigkeit während der andere Klebstoff durch Energiezufuhr reagiert.

5. Katheter nach Anspruch 4,
**dadurch gekennzeichnet, dass** der durch Zugabe von Feuchtigkeit reagierende Klebstoff ein Cyanacrylat ist.

6. Katheter nach Anspruch 4,
**dadurch gekennzeichnet, dass** der durch Energiezufuhr reagierende Klebstoff durch Bestrahlung mit ultraviolettem Licht aushärtbar ist.

## Claims

1. Catheter with a first component (1), a second component (2), and an adhesive join which is arranged between the components (1; 2) and which is filled by an adhesive seam (7) connecting the components (1; 2), **characterized in that** the adhesive seam (7) is configured in two layers, a first adhesive layer (8) being connected to the first component (1), and a second adhesive layer (9) being connected to the first adhesive layer (8) and to the second component (2).

2. Catheter according to Claim 1, **characterized in that** the adhesive layers (8; 9) are formed by chemically reacting adhesives.

3. Catheter according to Claim 2, **characterized in that** the adhesive layers (8; 9) are formed by chemically reacting adhesives with different reaction mechanisms.

4. Catheter according to Claim 3, **characterized in that** one adhesive reacts by addition of moisture, while the other adhesive reacts by supply of energy.

5. Catheter according to Claim 4, **characterized in that** the adhesive reacting by addition of moisture is a cyanoacrylate.

6. Catheter according to Claim 4, **characterized in that** the adhesive reacting by supply of energy can be hardened by irradiation with ultraviolet light.

## Revendications

1. Cathéter avec un premier composant (1), un deuxième composant (2) et une fente de collage arrangée entre les composants (1; 2), laquelle est remplie d'une couche d'adhésif (7) liant les composants (1; 2), **caractérisé en ce que** la couche d'adhésif (7) est formée en deux couches, une première couche d'adhésif (8) étant liée au premier composant (1) et une deuxième couche d'adhésif (9) étant liée à la première couche d'adhésif (8) et au deuxième composant.

2. Cathéter selon la revendication 1, **caractérisé en ce que** les couches d'adhésif (8, 9) sont formés d'adhésifs réagissant chimiquement.

3. Cathéter selon la revendication 2, **caractérisé en ce que** les couches d'adhésif (8, 9) sont formés d'adhésifs réagissant chimiquement avec des mécanismes de réaction différents.

4. Cathéter selon la revendication 3, **caractérisé en ce qu'**un adhésif réagit par addition d'humidité tandis que l'autre adhésif réagit par apport d'énergie.

5. Cathéter selon la revendication 4, **caractérisé en ce que** l'adhésif réagissant par addition d'humidité est un cyanoacrylate.

6. Cathéter selon la revendication 4, **caractérisé en ce que** l'adhésif réagissant par apport d'énergie est durcissable par exposition à la lumière ultraviolette.
